(19) Europäisches Patentamt
European Patent Office
Office européen des brevets

(11) EP 1 824 908 B1

(12) **EUROPÄISCHE PATENTSCHRIFT**

(45) Veröffentlichungstag und Bekanntmachung des Hinweises auf die Patenterteilung:
**19.01.2011 Patentblatt 2011/03**

(21) Anmeldenummer: **05815756.1**

(22) Anmeldetag: **28.11.2005**

(51) Int Cl.:
***C08J 3/20*** (2006.01) ***C08F 120/00*** (2006.01)
***A61L 15/16*** (2006.01) ***A61L 15/18*** (2006.01)
***A61L 15/24*** (2006.01) ***C08J 3/12*** (2006.01)

(86) Internationale Anmeldenummer:
**PCT/EP2005/012679**

(87) Internationale Veröffentlichungsnummer:
**WO 2006/058683 (08.06.2006 Gazette 2006/23)**

(54) **UNLÖSLICHE METALLSULFATE IN WASSERABSORBIERENDEN POLYMERPARTIKELN**

INSOLUBLE PETAL SULPHATES CONTAINED IN WATER-ABSORBING POLYMER PARTICLES

SULFATES METALLIQUES INSOLUBLES CONTENUS DANS DES PARTICULES POLYMERES ABSORBANT L'EAU

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.11.2004 DE 102004057868**

(43) Veröffentlichungstag der Anmeldung:
**29.08.2007 Patentblatt 2007/35**

(73) Patentinhaber: **BASF SE**
**67056 Ludwigshafen (DE)**

(72) Erfinder:
- **RIEGEL, Ulrich**
  **66849 Landstuhl (DE)**
- **DANIEL, Thomas**
  **67165 Waldsee (DE)**
- **HERMELING, Dieter**
  **67459 Böhl-Iggelheim (DE)**
- **ELLIOTT, Mark**
  **67063 Ludwigshafen (DE)**

(56) Entgegenhaltungen:
**EP-A1- 0 621 041** **EP-A1- 1 457 541**
**WO-A1-2004/011048** **DE-A1- 4 214 334**

EP 1 824 908 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft wasserabsorbierende Polymerpartikel, enthaltend unlösliche Metallsulfate, sowie ein Verfahren zu ihrer Herstellung und ihre Verwendung.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie beispielsweise Flüssigkeitsweiterleitung (SFC) in der Windel und Absorption unter Druck (AUL), werden wasserabsorbierende Polymere im allgemeinen oberflächen- oder gelnachvernetzt. Diese Nachvernetzung erfolgt bevorzugt in wässriger Gelphase oder als Nachvernetzung der gemahlenen und abgesiebten Polymerpartikel.

Unter Nachvernetzung wird die Gel- bzw. Nachvernetzung von wasserabsorbierenden Hydrogelen verstanden.

Dazu geeignete Vernetzer sind Verbindungen, die mindestens zwei Gruppen enthalten, die mit den Carboxylatgruppen des hydrophilen Polymeren kovalente Bindungen bilden können. Geeignete Verbindungen sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A 083 022, EP-A 543303 und EP-A 530 438). Als Vernetzer geeignete Polyamidoamine sind insbesondere in EP-A 349 935 beschrieben.

Desweiteren sind als Vernetzer in DE-A 198 07 502 2-Oxazolidon und dessen Derivate, in WO 03/031482 Morpholin-2,3-dion und dessen Derivate, in DE-A 198 54 573 2-Oxotetrahydro-1,3-oxazin und dessen Derivate, in DE-A 198 54 574 N-Acyl-2-Oxazolidone und in DE-A 198 07 992 Bis- und Poly-2-oxazolidinone als geeignete Vernetzer beschrieben.

Die ältere deutsche Patentanmeldung mit dem Aktenzeichen 103 34 584.1 beschreibt die Verwendung bicyclischer Amidacetale zur Nachvernetzung.

Die Patentanmeldung WO 02/060983 beschreibt Superabsorberpartikel, die mit einem wasserunlöslichen Metallphosphat nachbehandelt werden. Dabei werden Metallphosphatpartikel mit der Oberfläche der Superabsorberpartikel assoziert. Dadurch werden Superabsorber mit hoher Absorptionskapazität, verbesserten Flüssigkeitstransporteigenschaften und hoher Anquellgeschwindigkeit erhalten. Die Metallphosphatpartikel haben vorzugsweise eine mittlere Teilchengröße von 2 bis 7 μm, d.h. durch Abrieb der Metallphosphatpartikel entsteht ein nicht unerheblicher Feinstaubanteil.

Die ältere deutsche Patentanmeldung mit dem Aktenzeichen 10 2004 015 686.7 offenbart die Herstellung feinteiliger und hochpermeabler Hydrogele. Dabei werden nachträglich Kalziumphosphatpartikel auf das Hydrogel aufgebracht und unter Zusatz dendritischer Polymere fixiert.

Aufgabe der vorliegenden Erfindung war es daher wasserabsorbierende Polymerpartikel bereitzustellen mit hoher Flüssigkeitsweiterleitung (Permeabilität) im gequollenen Zustand, die nicht stauben, die bei ihrer Herstellung keine speziellen Apparaturen zum Aufbringen der Phosphatschicht benötigen sowie keine teuren Hilfsstoffe erfordern.

Diese Aufgabe wurde gelöst durch Zugabe wasserunlöslicher Metallsulfate, insbesondere Kalziumsulfat, in die Monomerlösung vor der Polymerisation oder durch Einmischen in die Reaktionsmasse während der Reaktion. Die Zugabe kann sowohl als Pulverdosierung in einem geeigneten Mischorgan oder vorzugsweise als wässrige Dispersion des Sulfats erfolgen. Statt der reinen Sulfate können auch deren Hydrate oder beliebige Gemische aus diesen Komponenten verwendet werden. Erfindungsgemäß wird nur so viel Sulfat zugesetzt, dass zwar eine deutliche Steigerung der Flüssigkeitsweiterleitung erreicht wird, aber die Absorptionskapazität des Superabsorbers nicht wesentlich herabgesetzt wird. Setzt man zuviel Sulfat hinzu, so wird das wasserabsorbierende Polymer unnötig verdünnt und damit die Absorptionskapazität gesenkt, was normalerweise nicht gewünscht wird.

Wasserunlöslich bedeutet eine Löslichkeit von weniger als 2 g, vorzugsweise von weniger als 0,1 g, besonders bevorzugt von weniger als 0,01 g, in 100 ml Wasser bei 25°C.

Die so hergestellten wasserabsorbierenden Polymere weisen höhere Zentrifugenretentionskapazitäten (CRC), höhere Absorptionen unter Druck (AUL 0.7 psi) und insbesondere höhere Flüssigkeitweiterleitungen (SFC) auf als das sonst identische Vergleichspolymere ohne Sulfat. Weiterhin wurde gefunden, dass die benötigte Verweilzeit der sulfathaltigen Polymere in der Nachvernetzung im Vergleich zum ansonsten identischen Vergleichspolymer deutlich verkürzt ist.

Dies bedeutet, dass mit dem erfindungsgemäßen Verfahren höhere Durchsätze möglich sind und trotzdem wasserabsorbierende Polymere mit verbesserter Qualität erzeugt werden können.

Die erfindungsgemäßen wasserabsorbierenden Polymere können durch Polymerisation einer Mischung aus

a) mindestens einem ethylenisch ungesättigten, säuregruppentragenden Monomeren, wobei die Säuregruppen

zumindest teilweise, beispielsweise zu 5 bis 100 mol-% neutralisiert sein können,

b) mindestens einem Vernetzer,

c) gegebenenfalls einem oder mehreren mit a) copolymerisierbaren ethylenisch und/oder allylisch ungesättigten Monomeren und

d) gegebenenfalls einem oder mehreren wasserlöslichen Polymeren, auf die die Monomere a), b) und ggf. c) zumindest teilweise aufgepfropft werden können,

wobei das dabei erhaltene Grundpolymer A gegebenenfalls nachneutralisiert, so dass insgesamt 25 bis 100 mol-%, bevorzugt 65 bis 90 mol-% der Säuregruppen neutralisiert sind, getrocknet, klassiert und mit

e) mindestens einem Nachvernetzer,

nachbehandelt wird, hergestellt werden.

Das wasserunlösliche Metallsulfat kann in Substanz als Pulver oder als wässrige Dispersion vor der Polymerisation der Monomerlösung, d.h. bevor in der Monomerlösung gezielt Startradikale erzeugt werden, oder während der Polymerisation, d.h. bei einem Monomerumsatz von höchstens 90%, vorzugsweise höchstens 70%, besonders bevorzugt höchstens 50%, zugesetzt werden.

Wird eine wässrige Dispersion verwendet, so beträgt die Konzentration an Metallsulfat in der Dispersion typischerweise 1 bis 70 Gew.-%, vorzugsweise 5 bis 60 Gew.-%, besonders bevorzugt 10 bis 50 Gew.-%, ganz besonders bevorzugt 20 bis 40 Gew.-%.

Vorzugsweise wird das unlösliche Metallsulfat in der Nähe der Peakmaximumtemperatur zugesetzt. Dies bedeutet bei diskontinuierlichen Verfahren die Zugabe erfolgt typischerweise höchstens 15 Minuten, vorzugsweise höchstens 10 Minuten, besonders bevorzugt höchstens 5 Minuten, vor oder nach dem Zeitpunkt, an dem die Polymerisationstemperatur ihr Maximum erreicht. Für kontinuierliche Verfahren gilt entsprechendes, d.h. die Zugabe erfolgt typischerweise höchstens 15 Verweilzeitminuten, vorzugsweise höchstens 10 Verweilzeitminuten, besonders bevorzugt höchstens 5 Verweilzeitminuten, vor oder hinter der Stelle, an dem die Polymerisationstemperatur ihr Maximum erreicht.

Das unlösliche Metallsulfat, vorzugsweise Kalziumsulfat, wird üblicherweise so dosiert, dass seine Menge, bezogen auf das wasserabsorbierende Polymer, weniger als 5 Gew.-%, vorzugsweise weniger als 3 Gew.-%, besonders bevorzugt weniger als 1,5 Gew.-%, ganz besonders bevorzugt 0,001 bis 0,75 Gew.-%, beträgt.

Im erfindungsgemäßen Verfahren einsetzbar sind auch die bekannten Hydrate des Kalziumsulfats sowie weitere wasserunlösliche Sulfate, beispielsweise Bariumsulfat.

Analog zu den wasserunlöslichen Sulfaten können auch eines oder mehrere wasserunlösliche Phosphate, beispielsweise Kalziumphosphat, eingesetzt werden. Wasserunlösliche Phosphate erhöhen ebenfalls die Flüssigkeitsweiterleinung (SFC). Weiterhin ist es möglich eines oder mehrere Sulfate zusammen mit einem oder mehreren Phosphaten zu verwenden.

Die im erfindungsgemäßen Verfahren herstellbaren hydrophilen, hochquellfähigen Hydrogele (Grundpolymer A) sind insbesondere Polymere aus vernetzten (co)polymerisierten hydrophilen Monomeren, Polyasparaginsäure, Pfropf (co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Bevorzugt handelt es sich bei dem zu vernetzenden Polymer um ein Polymer, das Struktureinheiten enthält, die sich von Acrylsäure oder deren Estern ableiten, oder die durch Pfropfcopolymerisation von Acrylsäure oder Acrylsäureestern auf eine wasserlösliche Polymermatrix erhalten wurden. Diese Hydrogele sind dem Fachmann bekannt und beispielsweise in der US 4 286 082, DE-C 27 06 135, US 4 340 706, DE-C 37 13 601, DE-C 28 40 010, DE-A 43 44 548, DE-A 40 20 780, DE-A 40 15 085, DE-A 39 17 846, DE-A 38 07 289, DE-A 35 33 337, DE-A 35 03 458, DE-A 42 44 548, DE-A 42 19 607, DE-A 40 21 847, DE-A 38 31 261, DE-A 35 11 086, DE-A 31 18 172, DE-A 30 28 043, DE-A 44 18 881, EP-A 801 483, EP-A 455 985, EP-A 467 073, EP-A 312 952, EP-A 205 874, EP-A 499 774, DE-A 26 12 846, DE-A 40 20 780, EP-A 205 674, US 5 145 906, EP-A 530 438, EP-A 670 073, US 4 057 521, US 4 062 817, US 4 525 527, US 4 295 987, US 5 011 892, US 4 076 663 oder US 4 931 497 beschrieben.

Zur Herstellung dieser quellbaren hydrogelbildenden Polymere geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide sowie die Alkalimetall- und/oder Ammoniumsalze der Säuregruppen enthaltenden Monomeren. Des weiteren eignen sich wasserlösliche N-Vinylamide wie N-Vinylformamid oder auch Diallyldimethyl-ammoniumchlorid. Bevorzugte hydrophile Monomere sind Verbindungen der allgemeinen Formel I

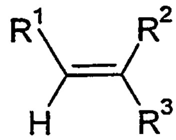

(I)

worin

$R^1$ Wasserstoff, $C_1$-$C_4$-Alkyl, wie beispielsweise Methyl oder Ethyl, oder Carboxyl,

$R^2$ -$COOR^4$, Hydroxysulfonyl oder Phosphonyl, eine mit einem $C_1$-$C_4$-Alkanol ver- esterte Phosphonylgruppe oder eine Gruppe der Formel II

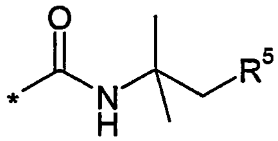

(II)

$R^3$ Wasserstoff, $C_1$-$C_4$-Alkyl, wie beispielsweise Methyl oder Ethyl,

$R^4$ Wasserstoff, $C_1$-$C_4$-Aminoalkyl, $C_1$-$C_4$-Hydroxyalkyl, Alkalimetall- oder Ammo- niumion und

$R^5$ eine Sulfonylgruppe, eine Phosphonylgruppe oder eine Carboxylgruppe oder jeweils deren Alkalimetall- oder Ammoniumsalze, bedeuten.

Beispiele für $C_1$-$C_4$-Alkanole sind Methanol, Ethanol, n-Propanol, Isopropanol oder n-Butanol.

Besonders bevorzugte hydrophile Monomere sind Acrylsäure und Methacrylsäure, sowie deren Alkalimetall- oder Ammoniumsalze, beispielsweise Natriumacrylat, Kaliumacrylat oder Ammoniumacrylat.

Geeignete Pfropfgrundlagen für hydrophile Hydrogele, die durch Pfropfcopolymerisation olefinisch ungesättigter Säuren oder ihrer Alkalimetall- oder Ammoniumsalze erhältlich sind, können natürlichen oder synthetischen Ursprungs sein. Beispiele sind Stärke, Cellulose oder Cellulosederivate sowie andere Polysaccharide und Oligo-saccharide, Polyalkylenoxide, insbesondere Polyethylenoxide und Polypropylenoxide, sowie hydrophile Polyester.

Geeignete Polyalkylenoxide haben beispielsweise die Formel III

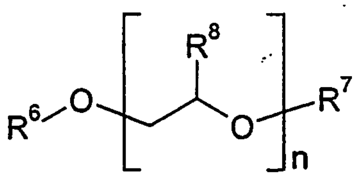

(III)

worin

$R^6$, $R^7$ unabhängig voneinander Wasserstoff, $C_1$-$C_{12}$-Alkyl, wie beispielsweise Methyl Ethyl, n-Propyl oder Isopropyl, $C_2$-$C_{12}$-Alkenyl, wie beispielsweise Ethenyl, n-Propenyl oder Isopropenyl, $C_7$-$C_{20}$-Aralkyl, wie beispielsweise Phenylmethyl, 1-Phenylethyl oder 2-Phenylethyl, oder Aryl, wie beispielsweise 2-Methylphenyl, 4-Methylphenyl oder 4-Ethylphenyl,

$R^8$ Wasserstoff oder Methyl und

n eine ganze Zahl von 3 bis 10000 bedeuten.

$R^6$ und $R^7$ bedeuten bevorzugt Wasserstoff, $C_1$-$C_4$-Alkyl, $C_2$-$C_6$-Alkenyl oder Phenyl.

Bevorzugte Hydrogele sind insbesondere Polyacrylate, Polymethacrylate sowie die in der US 4,931,497, US 5,011,892 und US 5,041,496 beschriebenen Pfropfpolymere.

Die quellbaren hydrogelbildenden Polymere sind bevorzugt vernetzt, d.h. sie enthalten Verbindungen mit mindestens zwei Doppelbindungen, die in das Polymernetzwerk einpolymerisiert sind. Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester; Polyallylester, Tetraallyloxyethan, Triallylamin, Tetraallylethylendiamin, Allylester der Phosphorsäure sowie Vinylphosphonsäurederivate, wie sie beispielsweise in EP-A 343 427 beschrieben sind. Besonders geeignete Vernetzer sind Di- und Triacrylate von mehrfach ethoxyliertem Glyzerin, Trimethylolpropan oder Trimethylolethan sowie deren korrespondierende Michaeladdukte. Beispielsweise kann während der Synthese aus je einem Di-und einem Triacrylat ein Pentaacrylat entstehen welches allein oder in beliebigen Gemischen mit dem ursprünglichen Di- oder Triacrylat als Vernetzer eingesetzt werden kann. Bevorzugt sind Triacrylate des 3- bis 20-fach ethoxylierten Glycerins oder Trimethylolpropans.

Weiterhin einsetzbar im erfindungsgemäßen Verfahren sind auch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Ethylenglykoldiallylether, Glyceroldi- und Triallylether, Polyallylether auf Basis Sorbitol, sowie ethoxilierte Varianten davon.

Die bevorzugten Herstellverfahren für das im erfindungsgemäßen Verfahren einsetzbare Grundpolymer werden in "Modern Superabsorbent Polymer Technology", F.L. Buchholz and A.T. Graham, Wiley-VCH, 1998, Seiten 77 bis 84 beschrieben. Besonders bevorzugt sind Grundpolymere, die im Kneter, wie beispielsweise in WO 01/38402 und WO 02/32964 beschrieben, oder auf einem Bandreaktor, wie beispielsweise in EP-A 955 086, EP-A 1 097 946 und EP-A 228 638 beschrieben, hergestellt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere in Mengen von 0,001 bis 10 mol-%, vorzugsweise 0,01 bis 1 mol-% enthalten, ganz besonders bevorzugt sind jedoch Polymere, die durch radikalische Polymerisation erhalten wurden und bei denen ein mehrfunktioneller ethylenisch ungesättigter Radikalvernetzer verwendet wurde, der zusätzlich noch mindestens eine freie Hydroxylgruppe tragen kann (wie beispielsweise Pentaerythritoltriallylether, Trimethylolpropandiallylether, Glycerindiacrylat).

Die quellbaren hydrogelbildenden Polymere können durch an sich bekannte Polymerisationsverfahren hergestellt werden. Bevorzugt ist die Polymerisation in wässriger Lösung nach dem Verfahren der sogenannten Gelpolymerisation. Dabei werden beispielsweise 15 bis 50 gew.-%ige wässrige Lösungen eines oder mehrerer hydrophiler Monomere und gegebenenfalls einer geeigneten Pfropfgrundlage in Gegenwart eines Radikalinitiators, bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0 und 150°C, vorzugsweise zwischen 10 und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff und/oder Wasserdampf, ausgeführt werden. zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxoverbindungen wie $(NH_4)_2S_2O_8$ oder $K_2S_2O_8$ oder $H_2O_2$. Sie können gegebenenfalls in Kombination mit Reduktionsmitteln wie Natriumhydrogensulfit und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie Mannichaddukte aus Sulfinsäuren, Aldehyden und Aminoverbindungen, wie sie in der DE-A-13 01 566 beschrieben sind, verwendet werden. Durch mehrstündiges Nachheizen der Polymergele im Temperaturbereich 50 bis 130°C, vorzugsweise 70 bis 100°C, können die Qualitätseigenschaften der Polymere noch verbessert werden.

Die erhaltenen Gele werden beispielsweise zu 25 bis 100 mol-%, bevorzugt 50 und 90 mol-%, insbesondere zwischen 60 und 90 mol-%, ganz besonders bevorzugt zwischen 65 und 80 mol-% und zwischen 65 und 72 mol-%, bezogen auf eingesetztes Monomer neutralisiert, wobei die üblichen Neutralisationsmittel verwendet werden können, bevorzugt Alkalimetallhydroxide oder-oxide, besonders bevorzugt jedoch Natriumhydroxid, Natriumcarbonat und Natriumhydrogencarbonat. Der pH-Wert des neutralisierten Grundpolymers beträgt üblicherweise zwischen 4,5 und 7,5, vorzugsweise zwischen 5,6 und 6,2.

Üblicherweise wird die Neutralisation durch Einmischung des Neutralisationsmittels als wässrige Lösung oder bevorzugt auch als Feststoff erreicht. Die Neutralisation wird vorzugsweise vor der Polymerisation in der Monomerlösung durchgeführt. Es kann aber auch das Polymergel neutralisiert oder nachneutralisiert werden. Hierzu wird das Gel typischerweise mechanisch zerkleinert, beispielsweise mittels eines Fleischwolfes und das Neutralisationsmittel wird aufgesprüht, übergestreut oder aufgegossen, und dann sorgfältig untergemischt. Dazu kann die erhaltene Gelmasse noch

mehrmals zur Homogenisierung gewolft werden.

Die neutralisierte Gelmasse wird mit einem Band- oder Walzentrockner getrocknet bis der Restfeuchtegehalt vorzugsweise unter 15 Gew.-%, mehr bevorzugt unter 8 Gew.%, insbesondere bevorzugt unter 5 Gew.-% liegt. Das getrocknete Grundpolymer A wird hiernach gemahlen und gesiebt, wobei zur Mahlung üblicherweise Walzenstühle, Stiftmühlen oder Schwingmühlen eingesetzt werden können. Die Partikelgröße des gesiebten Grundpolymers A liegt vorzugsweise im Bereich 45 bis 1000 μm, besonders bevorzugt bei 45 bis 850 μm, ganz besonders bevorzugt bei 100 bis 800 μm und noch mehr bevorzugt bei 100 bis 700 μm. Weitere bevorzugte Korngrössen liegen im Bereich 100 bis 500 μm, 150 bis 600 μm, 300 bis 600 μm, kleiner als 600 μm, kleiner als 400 μm, besonders bevorzugt kleiner als 300 μm, und am meisten bevorzugt kleiner als 150 μm. In diesen Bereichen liegen mindestens 80%, bevorzugt mindestens 90% aller Partikel.

Geeignete Grundpolymere können auch mit den weiterhin offenbarten Herstellverfahren in EP-A 316 792, EP-A 400 283, EP-A 343 427, EP-A 205 674 und der DE-A 44 18 818 erhalten werden. Außerdem können auch Sprühpolymerisationsverfahren eingesetzt werden.

Am meisten bevorzugt sind die aus kontinuierlichen Kneter- und Bandpolymerisationsanlagen erhaltenen Grundpolymere A.

Der CRC-Wert [g/g] des Grundpolymers A kann nach den in der Beschreibung angegebenen Methoden gemessen werden und ist bevorzugt mindestens 27, insbesondere mindestens 29, besonders bevorzugt mindestens 31, und höchstens 45, bevorzugt höchstens 39.

Der AUL-0,3psi-Wert [g/g] des Grundpolymers A kann nach den in der Beschreibung angegebenen Methoden gemessen werden und ist typischerweise mindestens 9, bevorzugt mindestens 14, insbesondere mindestens 17, besonders bevorzugt mindestens 21, und höchstens 27, bevorzugt höchstens 23.

Die Nachvernetzung von quellbaren hydrogelbildenden Polymeren wird in der Regel so durchgeführt, dass eine Lösung des Oberflächennachvemetzers auf das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Bevorzugt ist das Aufsprühen einer Lösung des Vernetzers in Reaktionsmischern oder Misch- und Trocknungsanlagen, wie beispielsweise Lödige®-Mischer, BEPEX®-Mischer, NAUTA®-Mischer, SCHUGI®-Mischer, NARA®-Trockner und PROCESSALL®. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 60 bis 200°C, und besonders bevorzugt bei 130 bis 195°C. Die bevorzugte Verweilzeit bei dieser Temperatur im Reaktionsmischer oder Trockner beträgt unter 60, bevorzugt unter 30 Minuten, besonders bevorzugt unter 10 Minuten.

Die Oberflächennachvernetzer können allein oder in Kombination mit anderen Oberflächennachvernetzem verwendet werden, beispielsweise Ethylenglykoldiglycidylether, Diethylenglykoldiglycidylether, Polyethylenglykoldiglycidylether, Propylenglykoldiglycidylether, Dipropylenglykoldiglycidylether, Polypropylenglykoldiglycidylether, Glycerindiglycidylether, Polyglycerindiglycidylether, Epichlorhydrin, Etylendiamin, Ethylenglykol, Diethylenglykol, Triethylenglykol, Polyethylenglykol, Propylenglykol, Dipropylenglykol, Tripropylenglykol, Polypropylenglykol, Butylenglykol, 1,3-Propandiol, 2-Methyl-1,3-Propandiol, 1,4-Butandiol, Bisphenol A, Glycerin, 1,5-Pentandiol, 1,6-Hexandiol, Neopentylglykol, Trimethylolpropan, Pentaerythrit, Sorbitol, Diethanolamin, Triethanolamin, Etylendiamin, Ethylencarbonat, Propylencarbonat, 2-Oxazolidone, wie 2-Oxazolidinon oder N-Hydroxyethyl-2-oxazolidinon, Morpholin-2,3-dione, wie N-2-Hydroxyethyl-morpholin-2,3-dion, N-Methyl-morpholin-2,3-dion , N-Ethyl-morpholin-2,3-dion und/oder N-tert.-Butyl-morpholin-2,3-dion, 2-Oxotetrahydro-1,3-oxazin, N-Acyl-2-oxazolidone, wie N-Acetyl-2-oxazolidon, bicyclische Amidacetale, wie 5-Methyl-1-aza-4,6-dioxa-bicyclo[3.3.0]octan, 1-Aza-4,6-dioxa-bicyclo[3.3.0]octan und/oder 5-Isopropyl-1-aza-4,6-dioxa-bicyclo[3.3.0]octan, und/oder Bis- und Poly-2-oxazolidinone.

Der Oberflächennachvernetzer wird bevorzugt in, bei gegebener Prozesstemperatur, nicht selbst-reaktiven Lösemitteln gelöst, bevorzugt in niederen Alkoholen, wie beispielsweise Methanol, Ethanol, Isopropanol, Propylenglykol, Ethylenglykol, vorzugsweise Isopropanol, ganz besonders bevorzugt in wässrigen Lösungen solcher geeigneter Alkohole, wobei der Alkoholgehalt der Lösung 10 bis 90 Gew.-%, besonders bevorzugt zwischen 25 bis 70 Gew.-%, insbesondere zwischen 30 bis 50 Gew.-% beträgt. Es können auch beliebige Gemische von Alkoholen eingesetzt werden.

Der Oberflächennachvernetzer wird dabei in einer Menge von 0,01 bis 1 Gew.-%, bezogen auf das eingesetzte Polymer, verwendet, und die Vernetzerlösung selbst in einer Menge von 1 bis 20 Gew.-%, bevorzugt 3 bis 15 Gew.-%, bezogen auf das eingesetzte Polymer, verwendet.

Bevorzugte Nachvemetzer sind 2-Oxazolidone, wie 2-Oxazolidinon oder N-Hydroxyethyl-2-oxazolidinon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxa-bicyclo[3.3.0]octan und/oder 5-Isopropyl-1-aza-4,6-dioxa-bicyclo[3.3.0]octan, Bis-2-oxazolidone und/oder Poly-2-oxazolidone.

Besonders bevorzugte Nachvemetzer sind 2-Oxazolidinon, N-Hydroxyethyl-2-oxazolidinon oder N-Hydroxypropyl-2-oxazolidinon.

Ein weiterer Gegenstand der vorliegenden Erfindung sind wasserabsorbierende Polymerpartikel, enthaltend

a) mindestens ein einpolymerisiertes ethylenisch ungesättigtes, säuregruppentragendes Monomer, wobei die Säuregruppen zumindest teilweise, beispielsweise zu 50 bis 85 mol-%, neutralisiert sein können,
b) mindestens einen einpolymerisierten Vernetzer,
c) gegebenenfalls ein oder mehrere mit a) copolymerisierbare einpolymerisierte ethylenisch und/oder allylisch ungesättigte Monomere,
d) gegebenenfalls ein oder mehrere wasserlösliche Polymere, auf die die Monomere a), b) und ggf. c) zumindests teilweise aufgepfropft sind,
e) mindestens einen umgesetzten Nachvernetzer und
f) mindestens ein einpolymerisiertes wasserunlösliches Metallsulfat.

Einpolymerisiert im Zusammenhang mit dem Metallsulfat bedeutet, dass das Metallsulfat in den Polymerpartikeln verteilt ist. Die Verteilung des Metallphosphats ist dabei vorzugsweise homogen oder weitgehend homogen. Im Gegensatz dazu stehen die Verfahren, wie sie beispielsweise in WO-A-02/60983 beschrieben sind, bei denen die Metallphospate auf die Polymerpartikel aufgesprüht werden und nur aufgrund von Adhäsionskräften auf der Oberfläche der Polymerpartikel haften.

Die Menge an Metallsulfat im wasserabsorbierenden Polymer beträgt weniger als 5 Gew.-%, vorzugsweise weniger als 3 Gew.-%, besonders bevorzugt weniger als 1,5 Gew.-%, ganz besonders bevorzugt 0,001 bis 0,75 Gew.-%. Bevorzugtes Metallsulfat ist Kalziumsulfat.

Bevorzugtes Monomer a) ist Acrylsäure, vorzugsweise zu 0 bis 100 mol-%, bevorzugt 5 bis 90 mol-%, insbesondere zwischen 25 und 80 mol%, ganz besonders bevorzugt zwischen 30 und 55 mol-% und zwischen 65 und 75 mol-%, bezogen auf eingesetztes Monomer, neutralisiert.

Der CRC-Wert [g/g] der erfindungsgemäßen nachvernetzten wasserabsorbierenden Polymere kann nach den in der Beschreibung angegebenen Methoden gemessen werden und ist bevorzugt mindestens 20, insbesondere mindestens 24, besonders bevorzugt mindestens 25, insbesondere mindestens 26, insbesondere bevorzugt mindestens 30.

Der AUL-0,7psi-Wert [g/g] der erfindungsgemäßen nachvernetzten wasserabsorbierenden Polymere kann nach den in der Beschreibung angegebenen Methoden gemessen werden und ist bevorzugt mindestens 15, insbesondere mindestens 21, besonders bevorzugt mindestens 22, insbesondere mindestens 23, insbesondere bevorzugt mindestens 25.

Der SFC-Wert [$10^{-7}$ $cm^3s/g$] der erfindungsgemäßen nachvernetzten wasserabsorbierenden Polymere kann nach den in der Beschreibung angegebenen Methoden gemessen werden und ist bevorzugt mindestens 30, insbesondere mindestens 45, besonders bevorzugt mindestens 60, insbesondere mindestens 70, insbesondere bevorzugt mindestens 80, und höchstens 1000.

Erfindungsgemäß bevorzugte Korngrößenbereiche sind 50 bis 700 μm, vorzugsweise 50 bis 500 μm oder 150 bis 700 μm, besonders bevorzugt 50 bis 400 μm oder 150 bis 600 μm, ganz besonders bevorzugt 50 bis 300 μm oder 150 bis 500 μm. In diesen Bereichen liegen mindestens 80 Gew.-%, bevorzugt mindestens 90 Gew.-%, und am meisten bevorzugt bis zu 100 Gew.-% aller Partikel.

Ein weiterer Gegenstand der vorliegenden Erfindung ist die Verwendung von Metallsulfaten, insbesondere Kalziumsulfat, bei der Herstellung von wasserabsorbierenden Polymeren vor oder während der Polymerisation.

Ein weiterer Gegenstand der vorliegenden Erfindung sind Hygieneartikel, wie Windeln, Tampons oder Damenbinden, insbesondere Windeln, enthaltend ein erfindungsgemäßes wasserabsorbierendes Polymer.

Zur Bestimmung der Güte der Nachvernetzung wird das getrocknete Hydrogel mit den Testmethoden geprüft, die nachfolgend beschrieben sind:

Methoden:

Die Messungen sollten, wenn nicht anders angegeben, bei einer Umgebungstemperatur von 23 ± 2 °C und einer relativen Luftfeuchte von 50 ± 10 % durchgeführt werden. Das quellbare hydrogelbildende Polymer wird vor der Messung gut durchmischt.

<u>Zentrifugenretentionskapazität (CRC Centrifuge Retention Capacity)</u>

Bei dieser Methode wird die freie Quellbarkeit des Hydrogels im Teebeutel bestimmt. Zur Bestimmung der CRC werden 0,2000 ± 0,0050 g getrocknetes Hydrogel (Kornfraktion 106 - 850 μm) in einem 60 x 85 mm großen

Teebeutel eingewogen, der anschließend verschweißt wird. Der Teebeutel wird für 30 Minuten in einen Überschuss von 0,9 gew.-%iger Kochsalzlösung gegeben (mindestens 0,83 l Kochsalzlösung/1 g Polymerpulver). Anschließend wird der Teebeutel 3 Minuten lang bei 250 G zentrifugiert. Die Bestimmung der vom Hydrogel festgehaltenen Flüssigkeitsmenge geschieht durch Auswägen des zentrifugierten Teebeutels.

Die Zentrifugenretentionskapazität kann auch nach der von der EDANA (European Disposables and Nonwovens Association) empfohlenen Testmethode Nr. 441.2-02 "Centrifuge retention capacity" bestimmt werden.

Absorption unter Druck (AUL Absorbency Under Load) 0,7 psi (4830 Pa)

Die Messzelle zur Bestimmung der AUL 0,7 psi ist ein Plexiglas-Zylinder mit einem Innendurchmesser von 60 mm und einer Höhe von 50 mm, der an der Unterseite einen angeklebten Edelstahl-Siebboden mit einer Maschenweite von 36 $\mu$m besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1344 g. Zur Durchführung der Bestimmung der AUL 0,7 psi wird das Gewicht des leeren Plexiglas-Zylinders und der Plastikplatte ermittelt und als W0 notiert. Dann werden 0,900 $\pm$ 0,005 g quellbares hydrogelbildendes Polymer (Komgrößenverteilung 150 - 800 $\mu$m) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als Wa notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm, einer Höhe von 10 mm und einer Porosität 0 gelegt und soviel 0,9 gew.-%ige Natriumchloridlösung eingefüllt, dass die Flüssigkeitsoberfläche mit der Filterplattenoberfläche abschließt, ohne dass die Oberfläche der Filterplatte benetzt wird: Anschließend wird ein rundes Filterpapier mit einem Durchmesser von 90 mm und einer Porengröße < 20 $\mu$m (S&S 589 Schwarzband von Schleicher & Schüll) auf die keramische Platte gelegt. Der quellbares hydrogelbildendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als Wb notiert.

Die Absorption unter Druck (AUL) wird wie folgt berechnet:

$$\text{AUL 0,7 psi [g/g]} = [Wb-Wa]/[Wa-W0]$$

Die Absorption unter Druck kann auch nach der von der EDANA (European Disposables and Nonwovens Association) empfohlenen Testmethode Nr. 442.2-02 "Absorption under pressure" bestimmt werden.

Absorption unter Druck (AUL Absorbency Under Load) 0,3 psi (2070 Pa)

Die Messung wird analog der AUL 0.7 psi durchgeführt. Das Gewicht der Plastikplatte und das Gewicht betragen zusammen 576 g.

Flüssigkeitsweiterleitung (SFC Saline Flow Conductivity)

Die Flüssigkeitsweiterleitung einer gequollenen Gelschicht unter Druckbelastung von 0,3 psi (2070 Pa) wird, wie in EP-A-0 640 330 beschrieben, als Gel-Layer-Permeability einer gequollenen Gelschicht aus superabsorbierendem Polymer bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A 640 330. Der Durchfluss wird automatisch erfasst.

Die Flüssigkeitsweiterleitung (SFC) wird wie folgt berechnet:

$$\text{SFC [cm}^3\text{s/g]} = (Fg(t=0)xL0)/(dxAxWP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$ darstellt.

Beispiele

Beispiel 1

In einen Lödige-Pflugscharkneter Typ VT 5R-MK (5 l Volumen) wurden 416 g Wasser, 189,5 g Acrylsäure, 1990,2 g einer 37,3 gew.-%igen Natriumacrylatlösung (100 mol% neutralisiert) sowie 4,55 g (= 0,60 Gew.% bezogen auf Acrylsäuremonomer) des Vernetzers Trimethylolpropan-15 EO-Triacrylat vorgelegt und unter Durchperlen von Stickstoff 20 Minuten inertisiert. Dann wurde durch Zusatz (verdünnte wässrige Lösungen) von 2,123 g Natriumpersulfat, 0,045 g Ascorbinsäure sowie 0,126 g Wasserstoffperoxid bei ca. 23 °C gestartet. Nach dem Start wurde die Temperatur des Heizmantels der Reaktionstemperatur im Reaktor mittels Regelung nachgeführt. Es wurde unter Rühren und guter Durchmischung im Kneter polymerisiert. Das letztlich erhaltene krümelige Gel wurde dann bei 180°C ca. 3 Stunden im Umlufttrockenschrank getrocknet. Anschließend wurde gemahlen und auf 200 bis 850 µm abgesiebt.

Der Ansatz wurde mehrmals wiederholt und die erhaltenen Pulver der einzelnen Ansätze vermischt und homogenisiert.

Das erhaltene Grundpolymer A wurde schließlich charakterisiert.

Die Eigenschaften des Grundpolymers A (200 bis 850 µm) waren wie folgt:

CRC = 35,6 g/g
AUL 0.3 psi = 14,3 g/g

Partikelgrößenverteilung des Grundpolymers A

| | |
|---|---|
| > 850 µm | = 12,75 Gew.-% |
| 600-850 µm | = 51,85 Gew.-% |
| 300-600 µm | = 30,46 Gew.-% |
| < 300 µm | = 4,95 Gew.-% |

In einem Lödige Labormischer wurden auf 1000 g Grundpolymer A, 2,898 Gew.-% Isopropanol/Wasser (Gewichtsverhältnis 30,8:69,2) und 0,085 Gew.-% 2-Oxazolidinon (25 gew.-%ige Lösung in 2:1-Gemisch Wasser/Propylenglykol), jeweils bezogen auf Grundpolymer, in einer Lösung aufgesprüht und das Polymer in einen zweiten, bereits vorgeheizten Lödige-Labormischer überführt und 120 Minuten bei 175°C Produkttemperatur nachvemetzt. Anschließend wurde das erhaltene Polymer bei 850 µm abgesiebt, um eventuell vorhandene Klumpen abzutrennen.

Die Nachvemetzungslösung hatte folgende Zusammensetzung: 0,85 g 2-Oxazolidon, 0,85 g Propylenglykol, 8,88 g Isopropanol und 21,8 g Wasser.

Das nachvernetzte, wasserabsorbierende Polymer hatte folgende Eigenschaften:

| Temperung | CRC [g/g] | AUL 0.7 psi [g/g] | SFC [$10^{-7}$ $cm^3$ s $g^{-1}$] |
|---|---|---|---|
| 70 Minuten | 30,1 | 24,0 | 26 |
| 120 Minuten | 28,9 | 21,7 | 46 |

Partikelgrößenverteilung nach 120 Minuten

| | |
|---|---|
| > 850 µm | = 0,96 Gew.-% |
| 600-850 µm | = 36,16 Gew.-% |
| 300-600 µm | = 51,39 Gew.-% |
| < 300 µm | = 11,49 Gew.-% |

Beispiele 2 bis 4

Es wurde analog zu Beispiel 1 ein Grundpolymer hergestellt, wobei die in der folgenden Tabelle angegeben Menge an Kalziumphosphat (Fa. Rhodia, Type TCP 130), bezogen auf als Acrylsäure berechnetes Monomer kurz vor Erreichen der Peaktemperatur der Polymerisation als 30 gew.-%ige wässrige Suspension zugesetzt wurde.
Das Grundpolymer wurde auf 200-850 μm abgesiebt und nachvernetzt.
Zur Nachvernetzung wurden 20 g des Grundpolymers in einem Waring-Labormischer mit einer Nachvemetzerlösung enthaltend 0,10 Gew.-% 2-Oxazolidon, 1,05 Gew.-% Isopropanol, 2,45 Gew-.% Wasser und 20 ppm Emulsogen V4345, jeweils bezogen auf eingesetztes Grundpolymer, unter Rühren besprüht und anschließend 60 Minuten bei 175°C im Umluftschrank getrocknet. Abschließend wurde bei 850 μm abgesiebt, um Klumpen zu entfernen.
Die Eigenschaften des Grund- und nachvemetzten Polymers sind wie folgt:

| Beispiel | Kalziumphosphat | CRC* [g/g] | AUL0.3psi * [g/g] | CRC** [g/g] | AUL0.7psi** [g/g] | SFC** [$10^{-7}$ $cm^3$ s $g^{-1}$] |
|---|---|---|---|---|---|---|
| 2 | 0,5 Gew.-% | 32,6 | 19,1 | 32,5 | 24,2 | 50 |
| 3 | 3,0 Gew.-% | 31,0 | 22,4 | 31,2 | 23,0 | 82 |
| 4 | 5,0 Gew.-% | 29,6 | 22,0 | 29,4 | 22,8 | 90 |
| *) Grundpolymer<br>**) nachvernetztes Polymer | | | | | | |

Beispiele 5 bis 7

Es wurde verfahren wie in den Beispielen 2 bis 4, nur dass Kalziumsulfat-Dihydrat (Fa. Merck, mindestens 99% rein) statt Kalziumphosphat zugesetzt wurde.
Die Eigenschaften des Grund- und nachvemetzten Polymers sind wie folgt:

| Beispiel | Kalziumsulfat Dihydrat | CRC* [g/g] | AUL0.3psi* [g/g] | CRC** [g/g] | AUL0.7psi** [g/g] | SFC** [$10^{-7}$ $cm^3$ s $g^{-1}$] |
|---|---|---|---|---|---|---|
| 5 | 0,5 Gew.-% | 32,1 | 23,4 | 32,2 | 24,3 | 80 |
| 6 | 3,0 Gew.-% | 31,0 | 22,3 | 31,2 | 23,2 | 57 |
| 7 | 5,0 Gew.-% | 30,1 | 21,3 | 31,0 | 22,0 | 37 |
| *) Grundpolymer<br>**) nachvernetztes Polymer | | | | | | |

## Patentansprüche

**1.** Verfahren zur Herstellung wasserabsorbierender Polymere durch Polymerisation einer Mischung aus

a) mindestens einem ethylenisch ungesättigten, säuregruppentragenden Monomeren, die zumindest teilweise neutralisiert sein können,
b) mindestens einem Vernetzer,
c) gegebenenfalls einem oder mehreren mit a) copolymerisierbaren ethylenisch und/oder allylisch ungesättigten Monomeren und
d) gegebenenfalls einem oder mehreren wasserlöslichen Polymeren, auf die die Monomere a), b) und ggf. c) zumindest teilweise aufgepfropft werden können,
wobei das dabei erhaltene Grundpolymer A mit
e) mindestens einem Nachvemetzer,

nachbehandelt wird, **dadurch gekennzeichnet, dass** man vor oder während der Polymerisation mindestens ein wasserunlösliches Metallsulfat zusetzt.

**2.** Verfahren gemäß Anspruch 1, **dadurch gekennzeichnet, dass** das Metallsulfat als Pulver dosiert wird.

3. Verfahren gemäß Anspruch 1, **dadurch gekennzeichnet, dass** das Metallsulfat als wässrige Dispersion dosiert wird.

4. Verfahren gemäß Anspruch 3, **dadurch gekennzeichnet, dass** die Konzentration an Metallsulfat in der Dispersion 1 bis 70 Gew.-% beträgt.

5. Verfahren gemäß einem der Ansprüche 1 bis 4, **dadurch gekennzeichnet, dass** das Metallsulfat in einer Menge von höchstens 5 Gew.-%, bezogen auf das wasserabsorbierende Polymer, eingesetzt wird.

6. Verfahren gemäß einem der Ansprüche 1 bis 5, **dadurch gekennzeichnet, dass** das Metallsulfat Kalziumsulfat ist.

7. Verfahren gemäß einem der Ansprüche 1 bis 6, **dadurch gekennzeichnet, dass** das Monomer a) eine teilneutralisierte Acrylsäure ist.

8. Verwendung von wasserunlöslichen Metallsulfaten zur Herstellung von wasserabsorbierenden Polymeren vor oder während der Polymerisation.

9. Wasserabsorbierende Polymerpartikel, enthaltend

a) mindestens ein einpolymerisiertes ethylenisch ungesättigtes, säuregruppentragendes Monomer, welches zumindest teilweise neutralisiert sein kann ,
b) mindestens einen einpolymerisierten Vernetzer,
c) gegebenenfalls ein oder mehrere mit a) copolymerisierbare einpolymerisierte ethylenisch und/oder allylisch ungesättigte Monomere,
d) gegebenenfalls ein oder mehrere wasserlösliche Polymere, auf die die Monomere a), b) und ggf. c) zumindests teilweise aufgepfropft sind,
e) mindestens einen umgesetzten Nachvernetzer und
f) mindestens ein einpolymerisiertes wasserunlösliches Metallsulfat,

wobei das mindestens eine Metallsulfat in den Polymerpartikein verteilt ist

10. Polymerpartikel gemäß Anspruch 9, enthaltend wasserunlösliches Metallsulfat in einer Menge von bis zu 5 Gew.-%, bezogen auf das wasserabsorbierende Polymer.

11. Polymerpartikel gemäß Anspruch 9 oder 10, wobei das Metallsulfat Kalziumsulfat ist.

12. Polymerpartikel gemäß einem der Ansprüche 9 bis 11, wobei das einpolymerisierte Monomer a) eine teilneutralisierte Acrylsäure ist.

13. Polymerpartikel gemäß einem der Ansprüche 9 bis 12, wobei die Polymerpartikel eine Flüssigkeitsweiterleitung von mindestens $30x10^{-7}$ $cm^3s/g$ aufweisen.

14. Polymerpartikel gemäß einem der Ansprüche 9 bis 12, wobei die Polymerpartikel eine Flüssigkeitsweiterleitung von mindestens $60x10^{-7}$ $cm^3s/g$ aufweisen.

15. Polymerpartikel gemäß einem der Ansprüche 9 bis 14, wobei die Polymerpartikel eine Zentrifugenretentionskapazität von mindestens 24 g/g aufweisen.

16. Hygieneartikel, enthaltend wasserabsorbierende Polymerpartikel gemäß einem der Ansprüche 10 bis 15.

## Claims

1. A process for producing water-absorbing polymers by polymerization of a mixture of

a) at least one ethylenically unsaturated acid-functional monomer which may each be at least partly neutralized,
b) at least one crosslinker,
c) optionally one or more ethylenically and/or allylically unsaturated monomers copolymerizable with a), and

d) optionally one or more water-soluble polymers which may be at least partially grafted with the monomers a), b) and if appropriate c),
the resulting base polymer A being aftertreated with
e) at least one postcrosslinker,

which comprises adding at least one water-insoluble metal sulfate before or during the polymerization.

2. The process according to claim 1 wherein the metal sulfate is metered as a powder.

3. The process according to claim 1 wherein the metal sulfate is metered as an aqueous dispersion.

4. The process according to claim 3 wherein the concentration of metal sulfate in the dispersion is in the range from 1% to 70% by weight.

5. The process according to any one of claims 1 to 4 wherein the metal sulfate is used in an amount of not more than 5% by weight, based on the water-absorbing polymer.

6. The process according to any one of claims 1 to 5 wherein the metal sulfate is calcium sulfate.

7. The process according to any one of claims 1 to 6 wherein the monomer a) is a partially neutralized acrylic acid.

8. The use of water-insoluble metal sulfates for producing water-absorbing polymers before or during the polymerization.

9. Water-absorbing polymeric particles comprising

a) at least one interpolymerized ethylenically unsaturated acid-functional monomer which may each be at least partly neutralized,
b) at least one interpolymerized crosslinker,
c) optionally one or more interpolymerized ethylenically and/or allylically unsaturated monomers copolymerizable with a),
d) optionally one or more water-soluble polymers which may be at least partially grafted with the monomers a), b) and if appropriate c),
e) at least one converted postcrosslinker, and
f) at least one interpolymerized water-insoluble metal sulfate,

the at least one metal sulfate being distributed in the polymeric particles.

10. The polymeric particles according to claim 9 that comprise water-insoluble metal sulfate in an amount of up to 5% by weight, based on the water-absorbing polymer.

11. The polymeric particles according to claim 9 or 10 wherein the metal sulfate is calcium sulfate.

12. The polymeric particles according to any one of claims 9 to 11 wherein the interpolymerized monomer a) is a partially neutralized acrylic acid.

13. The polymeric particles according to any one of claims 9 to 12 wherein the polymeric particles have a saline flow conductivity of not less than $30x10^{-7}cm^3s/g$.

14. The polymeric particles according to any one of claims 9 to 12 wherein the polymeric particles have a saline flow conductivity of not less than $60x10^{-7}cm^3s/g$.

15. The polymeric particles according to any one of claims 9 to 14 wherein the polymeric particles have a centrifuge retention capacity of not less than 24 g/g.

16. Hygiene articles comprising water-absorbing polymeric particles according to any one of claims 10 to 15.

## Revendications

**1.** Procédé pour la préparation de polymères absorbant l'eau, par polymérisation d'un mélange de

a) au moins un monomère à insaturation éthylénique, portant des groupes acides, qui peut être au moins partiellement neutralisé,
b) au moins un agent de réticulation,
c) éventuellement un ou plusieurs monomères à insaturation éthylénique et/ou allylique, copolymérisables avec a) et
d) éventuellement un ou plusieurs polymères hydrosolubles, sur lesquels peuvent être au moins partiellement greffés les monomères a), b) et éventuellement c),
le polymère de base A ainsi obtenu étant post-traité par
e) au moins un agent de post-réticulation,

**caractérisé en ce qu'**avant ou pendant la polymérisation on ajoute au moins un sulfate métallique insoluble dans l'eau.

**2.** Procédé selon la revendication 1, **caractérisé en ce que** le sulfate métallique est ajouté sous forme de poudre.

**3.** Procédé selon la revendication 1, **caractérisé en ce que** le sulfate métallique est ajouté sous forme de dispersion aqueuse.

**4.** Procédé selon la revendication 3, **caractérisé en ce que** la concentration de sulfate métallique dans la dispersion vaut de 1 à 70 % en poids.

**5.** Procédé selon l'une quelconque des revendications 1 à 4, **caractérisé en ce que** le sulfate métallique est utilisé en une quantité d'au maximum 5 % en poids, par rapport au polymère absorbant l'eau.

**6.** Procédé selon l'une quelconque des revendications 1 à 5, **caractérisé en ce que** le sulfate métallique est le sulfate de calcium.

**7.** Procédé selon l'une quelconque des revendications 1 à 6, **caractérisé en ce que** le monomère a) est un acide acrylique partiellement neutralisé.

**8.** Utilisation de sulfates métalliques insolubles dans l'eau, pour la préparation de polymères absorbant l'eau, avant ou pendant la polymérisation.

**9.** Particules de polymère absorbant l'eau, contenant

a) au moins un monomère à insaturation éthylénique, portant des groupes acides, qui peut être au moins partiellement neutralisé,
b) au moins un agent de réticulation incorporé par polymérisation,
c) éventuellement un ou plusieurs monomères à insaturation éthylénique et/ou allylique incorporés par polymérisation, copolymérisables avec a),
d) éventuellement un ou plusieurs polymères hydrosolubles, sur lesquels sont au moins partiellement greffés les monomères a), b) et éventuellement c),
e) au moins un agent de post-réticulation ayant réagi
et
f) au moins un sulfate métallique insoluble dans l'eau, incorporé par polymérisation,

ledit au moins un sulfate métallique étant réparti dans les particules de polymère.

**10.** Particules de polymère selon la revendication 9, contenant du sulfate métallique insoluble dans l'eau en une quantité allant jusqu'à 5 % en poids, par rapport au polymère absorbant l'eau.

**11.** Particules de polymère selon la revendication 9 ou 10, dans lesquelles le sulfate métallique est le sulfate de calcium.

**12.** Particules de polymère selon l'une quelconque des revendications 9 à 11, dans lesquelles le monomère a) incorporé

par polymérisation est un acide acrylique partiellement neutralisé.

**13.** Particules de polymère selon l'une quelconque des revendications 9 à 12, dans lesquelles les particules de polymère présentent une transmission de liquide d'au moins $30 \times 10^{-7} cm^3 s/g$.

**14.** Particules de polymère selon l'une quelconque des revendications 9 à 12, dans lesquelles les particules de polymère présentent une transmission de liquide d'au moins $60 \times 10^{-7} cm^3 s/g$.

**15.** Particules de polymère selon l'une quelconque des revendications 9 à 14, dans lesquelles les particules de polymère présentent un pouvoir de rétention centrifuge d'au moins 24 g/g.

**16.** Article d'hygiène, contenant des particules de polymère absorbant l'eau, selon l'une quelconque des revendications 10 à 15.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 083022 A
- EP 543303 A
- EP 530438 A
- EP 349935 A
- DE 198075022 A
- WO 03031482 A
- DE 198545732 A
- DE 19854574 A
- DE 19807992 A
- DE 10334584
- WO 02060983 A
- DE 102004015686
- US 4286082 A
- DE 2706135 C
- US 4340706 A
- DE 3713601 C
- DE 2840010 C
- DE 4344548 A
- DE 4020780 A
- DE 4015085 A
- DE 3917846 A
- DE 3807289 A
- DE 3533337 A
- DE 3503458 A
- DE 4244548 A
- DE 4219607 A
- DE 4021847 A
- DE 3831261 A
- DE 3511086 A
- DE 3118172 A
- DE 3028043 A
- DE 4418881 A
- EP 801483 A
- EP 455985 A
- EP 467073 A
- EP 312952 A
- EP 205874 A
- EP 499774 A
- DE 2612846 A
- EP 205674 A
- US 5145906 A
- EP 670073 A
- US 4057521 A
- US 4062817 A
- US 4525527 A
- US 4295987 A
- US 5011892 A
- US 4076663 A
- US 4931497 A
- US 5041496 A
- EP 343427 A
- WO 0138402 A
- WO 0232964 A
- EP 955086 A
- EP 1097946 A
- EP 228638 A
- DE 1301566 A
- EP 316792 A
- EP 400283 A
- DE 4418818 A
- WO 0260983 A
- EP 0640330 A
- EP 640330 A


### In der Beschreibung aufgeführte Nicht-Patentliteratur


- **F.L. Buchholz ; A.T. Graham.** Modern Superabsorbent Polymer Technology. Wiley-VCH, 1998, 77-84
- *Makromol. Chem.,* 1947, vol. 1, 169